Europäisches Patentamt

European Patent Office

Office européen des brevets

(11) Publication number: **0 148 131**
A1

## (12) EUROPEAN PATENT APPLICATION

(21) Application number: **84830206.3**

(22) Date of filing: **05.07.84**

(51) Int. Cl.⁴: **A 61 M 25/00**
**A 61 M 1/00**

(30) Priority: **13.07.83 IT 4868683**

(43) Date of publication of application:
**10.07.85 Bulletin 85/28**

(84) Designated Contracting States:
**AT BE CH DE FR GB LI LU NL SE**

(71) Applicant: **Pasqui, Ugo**
**Via Posillipo 9**
**Napoli(IT)**

(71) Applicant: **Cedrangolo, Laura**
**Via Posillipo 9**
**Napoli (Roma)(IT)**

(72) Inventor: **Pasqui, Ugo**
**Via Posillipo 9**
**Napoli(IT)**

(74) Representative: **Fiammenghi Domenighetti,**
**Delfina et al,**
**Via Quattro Fontane 31**
**I-00184 Rome(IT)**

(54) A cannula for the drainage of a vena cava.

(57) A cannula for the drainage of a vena cava (Cs, Ci), for establishing an extracorporeal blood circulation. Near a fluted end (3), this cannula (1) is provided with an expansion locking device (2), including an annular space (12) of variable volume, defined around the cannula wall (1), by a shaped sleeve (2) made of an elastically deformable material. The space (12) is connected by means of a thin tube (6) with a source of a pressurized fluid, so that, as the cannula (1) is inserted in a vena cava (Cs, Ci) and a fluid under pressure is fed in said annular space (12), this pressurized fluid causes the wall of the sleeve (2) to expand radially outwardly so as to seal press against the inner surface of the cannula (1). Thus the cannula (1) permits the by-passing of the heart and the total drainage of the venous blood from the respective vena cave (Cs, Ci) towards the cardio-pulmonary machine enabling an extracorporeal circulation, during surgical operations on opened heart.

EP 0 148 131 A1

./...

FIG. 4

0148131

A CANNULA FOR THE DRAINAGE OF A VENA CAVA FOR
ESTABLISHING AN EXTRACORPOREAL CIRCULATION, AND PROVIDED
WITH AN EXPANSION SEAL LOCKING DEVICE

DESCRIPTION

The present invention concerns the cannulae for the drainage of the venous blood from the venae cavae towards a machine for an extracorporeal blood circulation. During the surgical operations of the heart, it is absolutely necessary the use of a cardio-pulmonary machine, i.e. of an apparatus which is able to replace the functions of the heart and of the lungs of the patient during surgical operations on the heart.

In order to establish an extracorporeal blood circulation the venous blood of the patient is diverted from his venae cavae into the venous line of the cardio-pulmonary machine. The blood which is oxygenated inside this machine, is then fed again by this machine towards the arterial system of the blood circulation apparatus of the patient. Generally, it is necessary that all the venous blood is conveyed to the machine so as to leave the heart entirely without blood and therefore ready for any surgical operation on opened heart.

According to already widely tested surgical techniques, - one of which will be described thereinafter -, said drainage of the venous blood is, usually, obtained by

- 2 -                    0148131

means of the insertion of two cannulae or catheters in the superior and inferior vena cava respectively, each cannula passing through an incision made by the surgeon through the wall of the right atrium of the heart. The oxygenated blood is returned again in an artery of the patient through a third cannula.

The cannula or catheter for the venous blood hitherto used consists of a tube which may be less or more flexible, which is made of plastics and which has a straight axis or an axis bent at right angle; the distal end of this cannula which will be inserted into a vena cava, ends with a tip in the form of a flute mouth or of a wedge- -shaped extensively slotted tip. At the opposite end of the cannula, this latter is connected to the intake duct of an extracorporeal circulation apparatus. There are at present caval cannulae of different forms and dimensions, some of which are provided, in a portion of their wall, with a stainless steel wire helically wound round them, acting as reinforcing means. However, all the conventional cannulae or catheters for the drainage of the venous blood require the so called "operation of the binding of the venae cavae"; i.e. means and procedure are used, adapted to prevent that the venous blood can flow into the atrium. This operation consists in binding a ribbon or the like around each of the venous vessels, just upstream their outlet in the right atrium. By tightening or releasing the loop formed by each ribbon around the respective incannulated vena cava, it becomes thus possible to obtain a partial or total drainage of the venous blood, thus by-passing the heart.

More in particolar, the binding of the venae cavae requires a series of subsequent maneuvers, as will be thereinafter better described, with reference to the incannulation of the superior vena cava. The binding of the inferior vena cava is carried out in a similar manner.

By the use of a tampon at first the vena cava is sidely displaced and the auricle is lowered in order to explore the medial edge of the vena. By means of pliers the mesoderma of the vena cava will be then held tight at its postero-medial edge which is cut with the shears. By the use of a dissector gently maneuvered from the ouside towards the inside, the rear edge of the vena cava is cut down. The tip of the dissector is caused to come out medially in the previously cut point, so that the dissector can be passed round the vessel in the rear part thereof. By means of this dissector an end of the ribbon is grasped, so that, as the dissector is retracted, it can be let pass behind the vena cava. The maneuver for causing the ribbon slipping must be accompanied with the surgeon's fingers. The two ends of this ribbon is then joined one to the other, thus completing the binding of the superior vena cava. Subsequently the ribbon is inserted within a small tube of caoutchouc, this operation being necessary in order to enable to tighten or release the loop formed by the ribbon. Said ribbon insertion comprises the following steps: the laying of the two end portions of the ribbon on the longitudinal groove of a metal grooved guide having a width lightly lesser than the inner diameter of the small tube of caoutchouc designed to receive and restrain the ribbon end portion to be inserted into said tube; the sliding of said ribbon in the inside of said caoutchouc tube with the simultaneous retraction of said metal guide; and, at last, the anchoring of said ribbon end portions by small Klemmer pliers.

As has been already mentioned, a procedure similar to that which has been now described, must be followed for the binding of the inferior vena cava.

After having tied with ribbons both the venae cavae, a superior and an inferior caval cannulae are inserted by means of an atriotomy which is carried out, according to the known technique. By tightening more or less the ribbon loops, the already aforementioned total or partial drainage of the venous blood is carried out. From what has been set forth, it will be understood that, if a controlled drainage has to be obtained, using the cannulae available, at present, on the market, a tying round of each of the vena cava becomes absolutely necessary.

But that requires a non-negligible care from the surgical staff. Even if this operation procedure is carried out with skilfulness and care, there is a non-remote risk that such an operation may cause damages to the vena cava, which - on account of the fact that this latter has a thin wall -, could be injured , particularly in that part thereof which is hidden to the surgeon's view. Furthermore, if a new surgical operation on the heart should be required, the presence of the cicatricial scar could hinder the ribbon passage, that making this operation more dangerous. It has to be taken in due consideration, - particularly in the event of very urgent surgical operations -, the time required for carrying out said tying operation round the venae.

In order to overcome the aforementioned inconveniences the present invention provides a cannula for the caval drainage, which does not require such a tying operation. The cannula consists of a tube having the typical features of any known caval cannula, but which is provided, near the tip portion thereof, with an outer expansion locking device, including an inflatable annular cavity created between the outer surface of the cannula and the inner surface of a shaped sleeve made of an elastically deformable material and fixed, along its peripheral edges, to the outer surface of the cannula, said cavity being connected with an external source of a pressurized fluid by means of a thin pipe, the first length of which passes through the thickness of the cannula body in a longitudinal direction while the second length comes out from said wall.

Thus, after having inserted a cannula, according to the

- 6 -                    0148131

present invention, into the superior vena cava as well as another in the inferior one, following the known technique of the inoculation, it will be enough to supply the pressurized fluid in said annular space of the expansion locking device, in order to increase the volume of said annular space so that the sleeve inflates and expands radially thus creating a seal contact between the sleeve of the cannula and the inner surface of the vena cava. In this manner, by means of the deformation of the sleeve against the wall of the vena cava will be obtained the total drainage of the venous blood conveyed in the superior vena cava and into the inferior vena cava respectively, since any blowing -by of the blood in the right auricle about the cannula is prevented.

Owing to such a system of seal, pressing the inflatable sleeve against the inner surface of the vena wall, all the blood can be deviated into the respective cannula, duly controlling from the outside the pressure of the fluid supplied in the annular space defined between the cannula by means of an inflatable ballonet provided in the outer length of said thin pipe. Therefore, the fluid pressure acts in contrast with the pressure of the venous blood circulating in the cavae.

In contrast the seal connection obtained by the tying round the venae cavae of the prior art depends on the tension of the binding ribbon applied in the form of a loop, but this system is much more bloody, even if a total blood drainage is assured.

These and other characteristics and advantages of the
present invention will be better understood by the
following description of some embodiments of the
invention as well as by the method of use thereof,
reference being made to the accompanying drawings, in
which:

Figure 1 is a side view of a cannula for a caval
drainage, according to a first embodiment of the present
invention;

Figure 2 is a side view according to a second embodiment
of the cannula ;

Figure 3 is an enlarged cross section taken on the line
III-III of Figure 1, in the radially deformed condition
of the sleeve; and

Fig. 4 is a diagrammatical perspective view of the heart
and of the pericardial bed, which shows the incannulation
of each vena cava for a total blood drainage with the use
of cannulae according to the present invention.

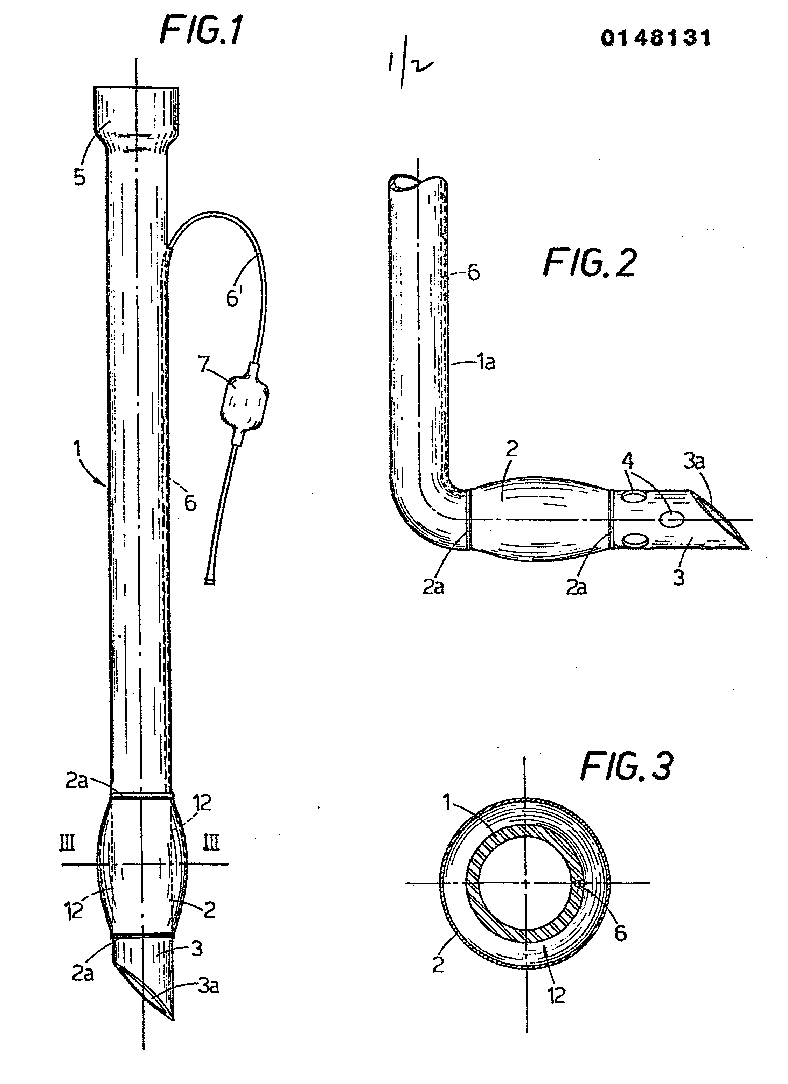

Now referring to Figures 1 to 3, 1 or 1a respectively
indicate a tube forming a cannula, and 2 is a sleeve
applied around the cannula 1 or 1a, so as to define
therebetween an annular cavity 12 of a variable volume .
According to a first embodiment, the tube 1 which is made of a semi-rigid plastic material compatible with the human organism, as, for instance, polyvinyl chloride, is straight. Its end portion 3, which is designed to be inserted into the vena cava has a tip 3a shaped as a flute mouth and obtained by means of a cut oblique to the axis of the tube 1. At the opposite tube end, the tube 1 is shaped as a female element 5 of a bell-and-spigot joint provided for the connection of the cannula 1 with conduit (not shown) of the venous line of a cardio- -pulmonary machine. Close enough to the tip 3a on the outer surface of the portion 3 of the cannula 1 a sleeve 2 is mounted made of an elastically deformable material, rubber, for instance. The peripheral edges 2a of said sleeve 2 are fixed by adhesive means to the outer surface of the tube 1 so as to define therebetween the space or chamber 12 of variable volume which is connected by means a thin pipe which comprises a first length which pass inside the thickness of the tube 1 in a longitudinal direction, and a second length 6a which travels out of the cannula 1, and in which a pressure gauge 7 is inserted, as for instance, an inflatable caoutchouc ballonet. The length 6a of said pipe 6, 6a will be connected with a source of a pressurized fluid, as for instance, a physiologic solution, since said fluid cannot cause any damage to the patient in the event of accidental breakages in any point of this small hydraulic plant.

As source of pressurized fluid a simple syringe is sufficient. After the injection of the fluid by means of a syringe, the outer length 6a of the pipe 6, 6a can be easily squeezed between the two arm elements of a clamp so as to stop any fluid return and prevent the deflation of said inner space 12. The inflation of the ballonet 7 indicates the inflation condition of the inner annular space 12 and thus the expanded condition of the sleeve 3. In Fig. 2 a second embodiment is shown of the cannula according to the present invention. In this case the tube 1a is bent at a substantially right angle. Its portion 3 comprised between the tip 3a and the sleeve 2 is provided with radial orifices 4 which are needed for increasing the blood inlet area. When cannulae 1a, bent at right angle, are employed, a superior atriotomy is necessary for the insertion of a cannula 1a into the superior vena cava $C_i$, and an inferior atriotomy for another cannula 1a for the inferior vena cava $C_i$. All the remaining features are similar to those of the first embodiment. If straight cannulae 1 are used, the contrary takes place, i.e. the two cannulae 1 cross into the atrium or auricle of the heart.

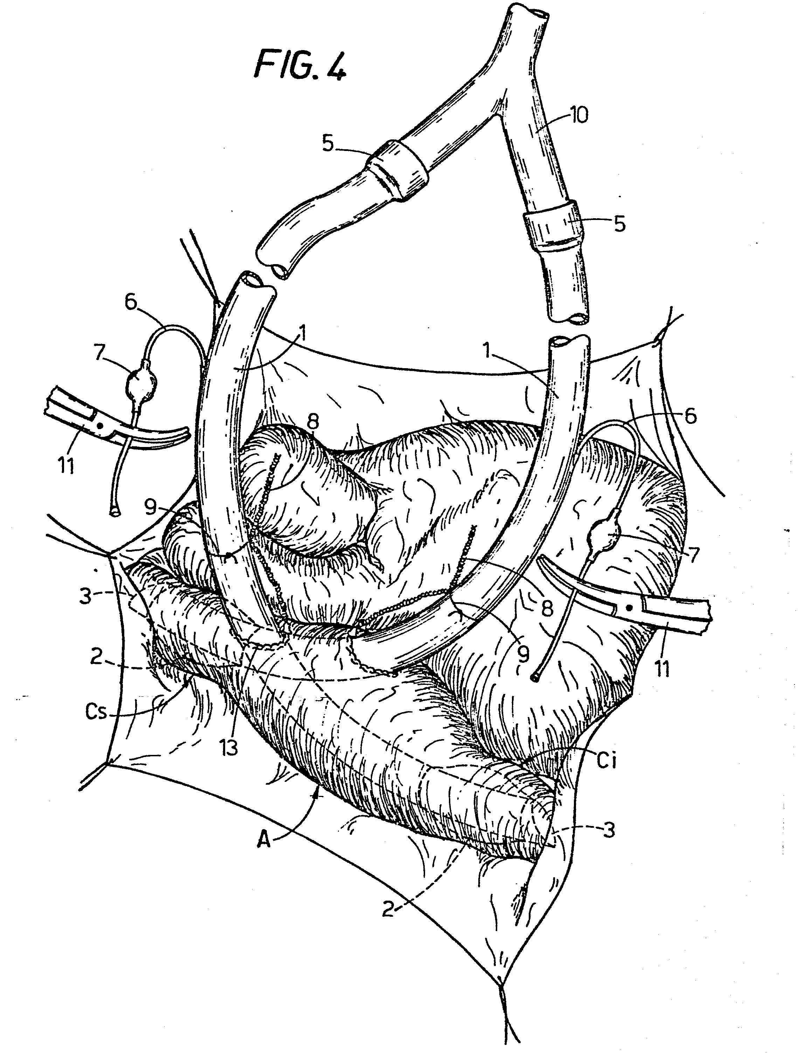

Figure 4 diagrammatically shows the use of two straight cannulae 1 constructed according to the first embodiment. In Figure 4, $C_s$ indicates the superior vena cava, $C_i$ the inferior vena cava and A is the atrium.

The present procedure for the incannulation will be now described with reference to the inferior vena cava $C_i$. By means of vessel pliers the atrial appendage is raised and is clamped near its base by an angiostate or vessel clamp. A purse-suture is obtained above the angiostate.

The ends of the thread 8 used to form the purse are locked into the clamping arms of a so called "tourniquet" or twisting device. Then the atrial appendage is grasped again with the vessel clamp and after having temporarily opened wide apart the arms of the angiostate, a dose of heparine is introduced in the area forming the purse. By the use of surgical shears, the atrial wall is incised in the area delimitated by said purse zone. The surgical orifice is then exposed, grasping the edges thereof by means of the arms of a vessel pliers. Now all is ready for the insertion of the cannula 1 into the atrium orifice 13. Thus the tip 3a of the cannula 1 is inserted in the cut 13 and then the end portion 3 of said cannula 1 is pushed forwards more and more, until said portion 3 with the sleeve 2, in deflated condition, attains its correct positioning, after having snap spread apart the angiostate.

At last the thread 8 defining the purse is pulled and twisted by the use of the tourniquet.

The cannula 1 is fixed to the tourniquet by means of a strong lace 9.

The incannulation of the superior vena cava $C_s$ is omitted, since it is substantially identical to the preceding one.

The two caval cannulae are connected by means of a rigid Y-pipe union 10 to the venous line of extracorporeal circuit of a cardio-pulmonary machine.

The annular spaces 12 of the sleeve devices are now inflated by the pressurized fluid and then the flexible pipe portion 6a is squeezed by means of a clamp 11. In this manner the walls of the sleeves 2 are elastically and radially deformed so as to sharply press against the caval walls. In this manner a seal contact between the caval wall and the respective cannula is attained and therefore the total deviation of the venous blood is obtained.

Before the extraction of the cannulae the clamps 11 are removed so that the flexible sleeves 2 of the expansion locking devices are deflated, by the discharge of the pressurized fluid out of the end of the pipe length 6a. For the decannulation of the inferior vena cava $C_1$ the lace 9 which fixes the cannula 1 is cut, while the angiostate is leaned against the atrium base.

Then the cannula 1 is quickly slipped out after having released the thread 8 of the purse-suture, while simultaneously the operator closes the surgical orifice 13 with the angiostate.

Then a light tension is imparted to the purse thread 8 and the surgical orifice 13 is furtherly sutured with an overcasting suture, tying in knot the ends of the suture thread. The base of the atrium is tied with a strong lace, progressively releasing the angiostate.

For the decannulation of the superior vena cava $C_s$ a similar procedure is followed.

Total drainage and by-pass tests carried out on animals have proved the high efficiency of the cannulae 1 o 1a, according to the present invention, which have been corrobotated by measurements of the venous pressure in

the superior and inferior venae cavae and by means of a supplementary atriotomy, which has permitted to directly observe the absence of any caval blood reflux into the atrium.

0148131

CLAIMS

1. A cannula for the drainage of the venae cavae for establishing an extracorporeal blood circulation, made of a semirigid plastic material, characterized by the fact that the cannula (1, 1a) has at one of its end (3) a tip (3a) shaped as a fluted mouth, and at the other end the female element (5) of a bell-and-spigot joint , and that at the end portion (3) of the cannula (1, 1a) near the tip (3a) thereof a sleeve (2) made of an elastically deformable material, rubber, for instance, is mounted around the cannula (1, 1a), said sleeve (2) being connected to the outer surface of the cannula (1, 1a) by fixing the annular edges (2a) of said sleeve (2) to the cannula wall by means of an adhesive or the like, so as to define therebetween an annular space (12) of variable volume, into which opens the orifice of a small pipe (6, 6a) the first length (6) of which passes longitudinally through the thickness of the cannula wall, while the second length (6a) thereof is positioned on the outside and includes an external pressure gauge (7), said pipe (6, 6a) being connected with a source of pressurized fluid which is supplied into the annular space (12) in such a quantity so as to increase the volume thereof and let expand the sleeve (2) so as to obtain a seal closure between the outer surface of the sleeve (2) and the inner surface of the vena cava ($C_s$ or $C_i$).

2. A cannula according to claim 1, wherein the cannula (1) has a straight axis.

3. A cannula according to claim 1, wherein the cannula has a curve axis, as for instance, bent to an angle of about 90°.

4. A cannula according to claim 1, wherein the cannula (1 or 1a) is provided in the part comprised between the tip (3a) and the expansion locking device (2, 12) of a plurality of orifices or slots (4).

5. A cannula according to claim 1, wherein the pressure gauge (7) is a ballonet, made of a material of a very high elasticity, as, for instance, rubber.

# FIG.1

# FIG.2

# FIG.3

FIG. 4

2/2          0148131

**European Patent Office**

**EUROPEAN SEARCH REPORT**

| | DOCUMENTS CONSIDERED TO BE RELEVANT | | | EP 84830206.3 |
|---|---|---|---|---|
| Category | Citation of document with indication, where appropriate, of relevant passages | | Relevant to claim | CLASSIFICATION OF THE APPLICATION (Int. Cl.4) |
| Y | US - A - 3 407 817 (E.A. GALLEHER) | | 1,2,4 | A 61 M 25/00 |
| A | * Totality * | | 5 | A 61 M 1/00 |
| | -- | | | |
| Y | US - A - 4 324 235 (A.V. BERAN) | | 1,2,4 | |
| A | * Totality * | | 5 | |
| | -- | | | |
| Y | US - A - 3 602 226 (R.E. ERICSON) | | 1,2,4 | |
| A | * Totality * | | 5 | |
| | -- | | | |
| Y | US - A - 3 890 976 (S. BAZELL et al.) | | 1,2,4 | |
| | * Totality * | | | |
| | -- | | | |
| Y | US - A - 3 734 100 (R.D. WALKER et al.) | | 1,2,3 | TECHNICAL FIELDS SEARCHED (Int. Cl.4) |
| | * Totality * | | | |
| | -- | | | A 61 M 1/00 |
| Y | GB - A - 1 186 964 (R.L. TINDEL) | | 1,2,3 | A 61 M 25/00 |
| A | * Totality * | | 5 | |
| | -- | | | |
| Y | GB - A - 1 058 888 (W. RÜSCH) | | 1,2,4 | |
| A | * Totality * | | 3,5 | |
| | -- | | | |
| Y | GB - A - 1 026 755 (F.E.B. FOLEY) | | 1,2,4 | |
| | * Totality * | | | |
| | -- | | | |

The present search report has been drawn up for all claims

| Place of search | Date of completion of the search | Examiner |
|---|---|---|
| VIENNA | 29-11-1984 | LUDWIG |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82

**0148131**
Application number

# EUROPEAN SEARCH REPORT

| | DOCUMENTS CONSIDERED TO BE RELEVANT | | EP 84830206.3 |
|---|---|---|---|

| Category | Citation of document with indication, where appropriate, of relevant passages | Relevant to claim | CLASSIFICATION OF THE APPLICATION (Int. Cl.4) |
|---|---|---|---|
| A | <u>GB - A - 2 047 538</u> (INT. PAPER COMP.)<br>* Fig. 2; page 2, line 99 *<br>-- | 1,2,4,5 | |
| A | <u>US - A - 3 971 385</u> (J.H. CORBETT)<br>* Fig. 1 *<br>-- | 1,2,4 | |
| A | <u>FR - A1 - 2 297 640</u> (RHONE POULENC IND.)<br>* Totality *<br>---- | | |
| | | | **TECHNICAL FIELDS SEARCHED (Int. Cl.4)** |

The present search report has been drawn up for all claims

| Place of search | Date of completion of the search | Examiner |
|---|---|---|
| VIENNA | 29-11-1984 | LUDWIG |